# EUROPEAN PATENT APPLICATION

(11) **EP 2 000 476 A2**
(43) Date of publication of application: **10.12.2008**
(21) Application number: 08154023.9
(22) Date of filing: 16.06.2005
(51) Int. Cl.: C07K 14/165, A61K 39/215

(54) **Immunogenic domains of SARS Coronavirus**

(30) Priority: 17.06.2004 US 580098 P
(62) Divisional of application: 05857456.7
(71) Applicant: Becton, Dickinson & Company, Franklin Lakes, NJ 07417-1880 (US)
(72) Inventor: Beadenkopf, Robert, J., Pasadena, MD 21122 (US)
(74) Representative: Helbing, Jörg

(57) **Abstract**

Potentially antigenic, conserved and specific SARS-CoV peptides are disclosed. The disclosed polypeptides may be used in a variety of diagnostic and treatment methods for SARS infection.

## Description

This application claims priority to U.S. Provisional Application Serial No. 60/580,098, filed June 17, 2004. This application is related to co-pending PCT International Patent Application Serial No. PCT/US04/029691, filed September 13, 2004, entitled "Assay for SARS Coronavirus by Amplification and Detection of the Replicase Sequence," and to co-pending PCT International Patent Application Serial No.PCT/US04/029692, also filed September 13, 2004, entitled "Assay for SARS Coronavirus by Amplification and Detection of Nucleocapsid RNA Sequence."

### Technical Field

The present invention relates to SARS Coronavirus (SARS-CoV) nucleocapsid, spike, envelope and membrane proteins. The peptides of the present invention have utility for diagnostics and therapy.

### Background Art

In the following discussion, certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Severe acute respiratory syndrome (SARS) is a recently emerging disease associated with atypical pneumonia in infected patients. The disease is unusually severe, and there is no known treatment. The incubation period for SARS is typically between 2 and to 10 days. Sympathkumar et al., Mayo Clin. Proc. 78: 882-890 (2003). Physical manifestations of SARS include fever, followed by a dry, nonproductive cough and shortness of breath. Death from respiratory failure occurs in about 3% to 10% of SARS cases. Centers for Disease Control and Prevention (CDC), Morb. Mortal. Wkly. Report. 52(16): 357 (2003).

Clinical diagnosis of SARS is often a slow process because initial diagnostic testing of suspected SARS patients includes a chest radiograph, pulse oximetry, blood culture, sputum Gram's stain and culture, and testing for other viral respiratory infections. CDC, *Guidelines and Recommendations: Interim Guidelines for Laboratory Diagnosis of SARS-CoV Infection* (Jul. 2003). As a specific example of the difficulties of diagnosis, one of the common diagnostic procedures-isolation in cell culture of SARS-CoV from a clinical specimen-often takes days or even weeks to complete. *Id.* The establishment of a rapid and noninvasive test for SARS-CoV is, therefore, essential for monitoring and control of the disease.

Early in 2003, a novel coronavirus was identified as the causative agent of SARS. Drosten et al., N. Engl. J. Med. 348: 1967-76 (2003). The coronaviruses are a diverse group of RNA viruses that cause respiratory and enteric diseases in humans and other animals. They are the largest of the RNA viruses, with a genome of approximately 30,000 nucleotides. The SARS-Coronavirus (SARS-CoV) is an enveloped, positive-stranded RNA virus. Based on sequence analysis, SARS-CoV is a member of a new group of coronavirus (order *Nidovirales,* Family *Coronaviridae,* genus *Coronavirus*). Rota et al., Science 300: 1394-1399 (2003). The genomic organization of SARS-CoV is similar to the other coronaviruses, with the gene order (5'-replicase [rep], spike [S], envelope [E], matrix [M], nucleocapsid [N]-3'). *Rota et al., supra.*

One important goal of SARS research is the identification of viral antigens that produce protective T-cell responses (useful candidates for the development of vaccines) or stimulate humoral immunity (important for diagnostic tools). The treatment and prevention of transmission of the SARS virus require reliable diagnostic tools to detect the antigens or antibodies relating to SARS. The nucleocapsid, spike, envelope, and matrix proteins provide valuable clinical targets for the detection and treatment of SARS infection.

### Disclosure of Invention

The present invention provides an isolated polypeptide encoded by a SARS-CoV gene, or a fragment or variant of said polypeptide, wherein the polypeptide comprises an amino acid sequence selected from the group consisting of SEQ ID NOs:1 through 27. In one embodiment, the polypeptide is produced by recombinant means. In a further embodiment, the invention provides a fusion protein comprising the isolated polypeptide of above. In an additional aspect, the present invention provides an isolated polypeptide comprising an amino acid sequence which is greater than about 90% identical to an amino acid sequence selected from SEQ ID NOs:1 through 27.

In a further aspect, the invention provides an antibody, or an antigen-binding fragment thereof, that selectively binds to an amino acid sequence selected from the group consisting of SEQ ID NOs:1 through 27, or to a fragment or variant of the amino acid sequence. In another embodiment, the antibody is a monoclonal antibody.

The present invention further provides a method of diagnosing SARS comprising detecting a polypeptide as described above in a test sample, wherein the presence of the polypeptide is indicative of the presence of SARS in the sample. In a further aspect, the method comprises contacting the test sample with an antibody specific for the polypeptide. In an additional aspect, the invention provides a method for assaying for the presence of the polypeptide in a sample, comprising contacting the sample with an antibody which specifically binds to the polypeptide.

In an additional embodiment, the present invention provides a pharmaceutical composition, comprising the polypeptide of claim 1, and a pharmaceutically acceptable carrier. In yet another embodiment, the method comprises administering an effective amount of the pharmaceutical composition described above to a patient in need thereof. The present invention further provides a kit, comprising: a) at least one antibody which selectively binds to an amino acid sequence selected from the group consisting of SEQ ID NOs:1 through 27; and b) a reference protein sample. In yet another embodiment, the present invention provides a kit, comprising a) at least one isolated polypeptide selected from the group consisting of SEQ ID NOs:1 through 27; and b) a reference protein sample.

In another aspect, the present invention provides a method of identifying an agent which alters activity of the polypeptide described previously comprising: a) contacting the polypeptide or fragment thereof with an agent to be tested; b) assessing the level of activity of the polypeptide or fragment thereof; and c) comparing the level of activity with the level of activity of the polypeptide or fragment thereof in the absence of the agent; wherein if the level of activity of the polypeptide or fragment thereof in the presence of the agent differs from the level in the absence of the agent, then the agent is an agent that alters activity of the peptide. The invention further provides an agent which alters the activity of a polypeptide of the invention, identifiable by the above method.

### Modes for Carrying Out the Invention

The present invention relates to amino acid sequences of SARS-CoV, the causative agent of SARS in humans. The invention involves in one aspect SARS-CoV polypeptides, as well as therapeutics relating thereto. The invention also embraces isolated functionally equivalent variants, useful analogs and fragments of the foregoing polypeptides, and antibodies and fragments thereof that selectively bind the foregoing polypeptides.

As used herein, the term "polypeptide" refers to a polymer of amino acids of a nonspecific length. The term, therefore, also encompasses proteins. The SARS-CoV polypeptides of the invention are isolated. As used herein, "isolated" means separated from its native environment and present in sufficient quantity to permit its identification or use. For example, polypeptides of the present invention that have been: (i) selectively produced by expression cloning or (ii) purified as by chromatography or electrophoresis are isolated, as that term is used herein. Isolated polypeptides may be, but need not be, substantially pure. The term "substantially pure" means that the polypeptides are essentially free of other substances with which they may be found in nature or in *in vivo* systems to an extent practical and appropriate for their intended use. Substantially pure polypeptides may be produced by techniques well known in the art. Because an isolated polypeptide may be admixed with a pharmaceutically acceptable carrier in a pharmaceutical preparation, the polypeptide may comprise only a small percentage by weight of the preparation. The polypeptide is nonetheless isolated in that it has been separated from the substances with which it may be associated in living systems, e.g., isolated from other polypeptides.

In one embodiment, a polypeptide comprises at least a portion of the amino acid sequence of at least one of SEQ ID NOs:1 through 27. The invention also includes functionally equivalent variants of the SARS-CoV polypeptides that retain one or more of the functional properties of the SARS-CoV polypeptides. Variants also include polypeptides substantially homologous or identical to at least a portion of at least one of SEQ ID NOs:1 thorough 27 but derived from another organism, produced by chemical synthesis or produced by recombinant methods. Other functionally equivalent variants will be known to one of ordinary skill in the art, as will methods for preparing such variants. Such variants are useful, *inter alia,* for evaluating bioavailability of drugs and in assays for identification of compounds that bind and/or regulate the function of the SARS-CoV polypeptides. Variants that are non-functional also can be prepared as described above. Such variants are useful, for example, as negative controls in experiments testing transporter activity.

The invention further includes fragments of the polypeptides of the invention. The fragments can be derived from the polypeptides of SEQ ID NOs:1 through 27 and comprise at least 10 contiguous amino acids. Preferably, the fragment retains at least one of the biological functions of the polypeptides of the invention. A fragment can be found within a larger non-related polypeptide or can be separate. The polypeptides of the invention may be fused to another polypeptide having an amino acid sequence that is not substantially homologous to SEQ ID NOs:1 through 27. As used in the present invention, two polypeptides are substantially homologous or identical when the amino acid sequences are preferably at least about 80-85% homologous or identical, more preferably at least about 90% homologous or identical. A purified polypeptide or fragment of the present invention may be isolated from cells in which it is naturally expressed or from recombinant cells or may be chemically synthesized.

The SARS-CoV polypeptides and fragments (including the SARS-CoV inhibitors described below) of the invention can be delivered to a eukaryotic or prokaryotic cell alone or in association with a vector. In its broadest sense, a "vector" is any vehicle capable of facilitating: (1) delivery of a SARS-CoV polypeptide to a target cell, (2) uptake of a SARS-CoV polypeptide by a target cell, or (3) expression of a SARS-CoV polypeptide in a target cell. Preferably, the vectors transport the SARS-CoV polypeptide into the target cell with reduced degradation relative to the extent of degradation of the polypeptide that would occur in the absence of the vector. Optionally, a "targeting ligand" can be attached to a vector to selectively deliver the vector to a cell that expresses on its surface the cognate receptor (e.g. a receptor, an antigen recognized by an antibody) for the targeting ligand. Examples of targeting ligands include an antibody fragment, a cytokine or a short peptide. In this manner, a vector (containing a SARS-CoV polypeptide) can be selectively delivered to a specific cell. In general, vectors useful in the present invention are divided into two classes: biological vectors and chemical/physical vectors.

Biological vectors include, but are not limited to, plasmids, phagemids, viruses and other vehicles derived from viral or bacterial sources that have been manipulated by the insertion or incorporation of nucleic acid sequences. As used herein, a "chemical/physical vector" refers to a natural or synthetic molecule, other than those derived from bacteriological or viral sources, capable of delivering the isolated SARS-CoV polypeptide to a cell.

The invention embraces variants of the SARS-CoV polypeptides described above. As used herein, a "variant" of a SARS-CoV polypeptide is a polypeptide that contains one or more modifications to the primary amino acid sequence of a SARS-CoV polypeptide. Modifications that create a SARS-CoV variant can be made to a SARS-CoV polypeptide for a variety of reasons, including (1) to reduce or eliminate an activity of a SARS-CoV polypeptide; (2) to enhance or an activity of a SARS-CoV polypeptide; (3) to provide a novel activity or property to a SARS-CoV polypeptide, such as addition of an antigenic epitope or addition of a detectable moiety; or (4) to establish whether an amino acid substitution affects the activity of the SARS-CoV polypeptide. Modifications can be made directly to the polypeptide, such as by cleavage, addition of a linker molecule, addition of a detectable moiety, such as biotin, addition of a fatty acid, and the like. Modifications also embrace fusion proteins comprising all or part of the SARS-CoV amino acid sequence. One of skill in the art will be familiar with methods for predicting the effect on protein conformation of a change in protein sequence, and can thus "design" a variant SARS-CoV according to known methods.

The skilled artisan will also realize that conservative amino acid substitutions may be made in SARS-CoV polypeptides to provide functionally equivalent variants of the foregoing polypeptides, i. e., variants that retain the functional capabilities of the SEQ ID NOs:1 through 27. As used herein, a "conservative amino acid substitution" refers to an amino acid substitution that does not alter the relative charge or size characteristics of the polypeptide in which the amino acid substitution is made. Variants can be prepared according to methods for altering polypeptide sequences known to one of ordinary skill in the art such as are found in references that compile such methods, *e.g.,* Molecular Cloning: A Laboratory Manual, J. Sambrook, et al., eds., Second Edition, Cold Spring Harbor Laboratory Press, Cold Spring Harbor, N.Y. (1989) or Current Protocols in Molecular Biology, F. M. Ausubel, et al., eds., John Wiley & Sons, Inc., New York. Exemplary functionally equivalent variants of the SARS-CoV polypeptides include conservative amino acid substitutions of SEQ ID NOs:1 through 27.

A variety of methodologies well known to the skilled practitioner can be utilized to obtain isolated SARS-CoV polypeptides. The polypeptide may be purified from cells that naturally produce the polypeptide, by chromatographic means or immunological recognition. Alternatively, an expression vector may be introduced into cells to cause production of the polypeptide. Those skilled in the art also can readily follow known methods for isolating SARS-CoV polypeptides. These include, but are not limited to, immunochromatography, HPLC, size-exclusion chromatography, ion-exchange chromatography and immune-affinity chromatography.

The invention also embraces agents that bind selectively to the SARS-CoV polypeptides, as well as agents that bind to variants and fragments of the polypeptides as described herein. The agents include antibodies that bind to SARS-CoV, anti-binding peptides and other molecules that bind to the SARS-CoV polypeptide and complexes containing the SARS-CoV polypeptide. The binding agents can inhibit or increase SARS-CoV activity (antagonists and agonists, respectively).

Some of the agents that bind SARS-CoV polypeptides are inhibitors. A SARS-CoV inhibitor is an agent that inhibits SARS-CoV polypeptide activity. Assays known to those of skill in the art can be performed to screen and/or determine SARS-CoV activity in the presence or absence of a candidate inhibitor. When the binding molecules are inhibitors, the molecules bind to and inhibit the activity of SARS-CoV.

Any binding assay known in the art may be employed to determine whether a SARS-CoV binding agent binds to SARS-CoV. For example, but not by way of limitation, the binding agent may be immobilized on a surface and then contacted with a labeled SARS-CoV polypeptide. The amount of SARS-CoV that interacts with the SARS-CoV binding agent or the amount that does not bind to the SARS-CoV binding agent may then be quantitated to determine whether the SARS-CoV binding agent binds to SARS-CoV.

The SARS-CoV binding agents include molecules of various size and type that bind selectively or preferentially to SARS-CoV polypeptides, and complexes of both SARS-CoV polypeptides and their binding partners. These molecules may be derived from a variety of sources. For example, SARS-CoV binding agents can be provided by screening degenerate peptide libraries that can be readily prepared in solution, in immobilized form, or as phage display libraries. Combinatorial libraries also can be synthesized from peptides containing one or more amino acids. Libraries of potential SARS-CoV binding molecules also can be synthesized from peptides and non-peptide synthetic moieties.

Phage display can be particularly effective in identifying binding peptides useful according to the present invention. Briefly, one prepares a phage library (using, e.g., m13, fd, or lambda phage), displaying inserts from 4 to about 80 amino acid residues using conventional procedures. The inserts may represent, for example, but not by way of limitation, a completely degenerate or biased array. One then can select phage-bearing inserts that bind to the SARS-CoV polypeptide. This process can be repeated through several cycles of reselection of phage that bind to the SARS-CoV polypeptide. Repeated rounds lead to enrichment of phage bearing particular sequences. DNA sequence analysis can be conducted to identify the sequences of the expressed polypeptides. The minimal linear portion of the sequence that binds to the SARS-CoV polypeptide can be determined. One can repeat the procedure using a biased library containing inserts containing part or the entire minimal linear portion plus one or more additional degenerate residues upstream or downstream thereof. Yeast two-hybrid screening methods also may be used to identify polypeptides that bind to the SARS-CoV polypeptides. Thus, the SARS-CoV polypeptides of the invention, or a fragment thereof, can be used to screen peptide libraries, including phage display libraries, to identify and select peptide-binding partners of the SARS-CoV polypeptides of the invention. Such molecules can be used, as described, for screening assays, for purification protocols, for interfering directly with the functioning of SARS-CoV and for other purposes that will be apparent to those of ordinary skill in the art upon combining knowledge in the art with the present disclosure.

The present invention, therefore, generally provides efficient methods of identifying pharmacological agents or lead compounds for agents useful in the treatment of conditions associated with SARS-CoV activity and the compounds and agents so identified. Generally, the screening methods involve assaying for compounds that inhibit or enhance transport of molecules through SARS-CoV. Such methods are adaptable to automated, high throughput screening of compounds.

A variety of assays for pharmacological agents are provided, including, *labeled in vitro* protein binding assays, efflux assays using detectable molecules, etc. For example, but not by way of limitation, protein-binding screens are used to rapidly examine the binding of candidate pharmacological agents to a SARS-CoV and to determine the effect of the agent on SARS-CoV function. The candidate pharmacological agents can be derived from, for example, but not by way of limitation, combinatorial peptide libraries.

SARS-CoV peptides used in the methods of the present invention can be added to an assay mixture as an isolated polypeptide (where binding of a candidate pharmaceutical agent is to be measured) or as a cell or other membrane-encapsulated space that includes a SARS-CoV polypeptide. In the latter assay configuration, the cell or other membrane-encapsulated space can contain the SARS-CoV as a preloaded polypeptide. The SARS-CoV polypeptide can be produced recombinantly, or isolated from biological extracts, or synthesized *in vitro.* SARS-CoV polypeptides encompass chimeric proteins comprising a fusion of a SARS-CoV polypeptide with another polypeptide, e.g., a polypeptide capable of providing or enhancing protein-protein binding, or enhancing stability of the SARS-CoV polypeptide under assay conditions. A polypeptide fused to a SARS-CoV polypeptide or fragment thereof may also provide means of readily detecting the fusion protein, e.g., by immunological recognition or by fluorescent labeling. Fluorescent, radioactive, or other recognition molecules may also be fused or otherwise attached directly to the SARS-CoV polypeptide.

These peptides of the present invention (e.g., SEQ ID NOs:1 through 27) can be synthesized relatively easily, at low cost, and at high purity. Conversely, production and purification of native pathogenic proteins through conventional means is often tedious and costly, requiring specialized facilities, expensive equipment and highly skilled personnel. Likewise, production and purification of complex foreign proteins through recombinant means is time consuming and also requires specialized skill resources. Consequently, antigenic peptide immunogens, like those produced from the peptides of the inventions (e.g., SEQ ID NOs:1 through 27) represent an attractive alternative compared to immunogens produced by conventional virological or recombinant means. The use of SARS-CoV specific antigenic peptide immunogens would eliminate the need to grow and purify the SARS-CoV virus in tissue culture in a BSL-3 lab. The use of the peptides of the present invention would eliminate the need to verify viral non-infectivity. Additionally, the use of disclosed peptides would eliminate the need to molecularly clone, express and purify SARS viral proteins.

The polypeptides of the present invention (e.g., SEQ ID NOs:1 through 27) can be used to raise antibodies or elicit an immune response. The term "antibody," as used herein, refers to immunoglobulin molecules and active portions of immunoglobulin molecules. These antibodies can be used to isolate or detect the presence of a polypeptide of the invention in a test sample. Any of the protocols well known in the art may be used to produce the antibodies of the invention *(see, e.g.,* Current Protocols in Immunology, Coligan et al. (eds.), John Wiley & Sons, Inc., New York, N.Y (1994)). For example, but not by way of limitation, polyclonal antibodies may be prepared by immunizing a suitable subject with the desired immunogen (e.g., a polypeptide of the invention).

Antigenic peptides inherent to complex foreign proteins, such as the peptides of the present invention, have several advantages over native proteins. The disclosed peptides make suitable immunogens alone, but may also be made more immunogenically complex. This may be done by conjugation to carrier proteins or conversion to a highly branched multi-peptide moiety and will typically impart enough complexity to the peptide to render it more capable of eliciting an immunogenic response when injected into an animal. For example, but not by way of limitation, the peptides of the invention (SEQ ID NOs:1 through 27) may be synthesized to a high level of purity using methods well known to those of skill in the art. One or more additional extraneous yet specific amino acids can be added to either the N or C-terminus for purposes of subsequent conjugation via a heterobifunctional reactant to either bovine serum albumin (BSA) or keyhole limpet hemocyanin (KLH). For each of the disclosed peptides produced, heterobifunctional reagent A can be used to conjugate the peptide to BSA, and heterobifunctional reagent B can be used to conjugate the peptide to KLH, thereby forming a conjugate pair. One conjugate serves as the immunogen, and the other serves as means of screening.

Many of the procedures well known in the art may be used to create monoclonal antibodies to a polypeptide of the invention (e.g., SEQ ID Nos:1 through 27). For example, antibody-secreting hybridomas may be produced using methods such as those set forth by Kohler and Milstein (Kohler and Milstein, Nature 256(5517): 495-7 (1975)) and/or variations of those methods, which are well known in the art. The human B-cell hybridoma technique is an additional exemplary hybridoma technique for making monoclonal antibodies. Cote et al., Proc. Natl. Acad. Sci. U S A. 80(7): 2026-30 (1983). As another example, monoclonal antibodies may be identified by screening recombinant combinatorial immunoglobulin libraries. Such libraries may be produced by many art-known methods, such as phage display *(see, e.g.,* McCafferty et al., Nature 348(6301): 552-4 (1990)), which is also described herein above.

Chimeric antibodies that specifically recognize peptides according to the present invention (e.g., SEQ. Nos:1 through 27) are also useful in therapeutic and diagnostic applications as described herein and may also be prepared by art-known methods, such as those described in Morrison et al., Proc Natl. Acad. Sci. U S A 81(21): 6851-5 (1984) and Takeda et al., Nature 314(6010):452-4 (1985). In general, chimeric antibodies are prepared by splicing the genes from a mouse antibody molecule of desired antigen specificity together with genes from a human antibody molecule of desired biological activity. Furthermore, single chain antibodies that specifically recognize peptides according to the present invention (*e.g.,* SEQ. ID Nos:1 through 27) are also useful in therapeutic and diagnostic applications as described herein and may also be prepared by art-lcnown methods, such as those described in Bird et al., Science 242(4877):423-6 (1988) and Huston et al., Proc Natl. Acad. Sci. U S A 85(16):5879-83 (1988). In general, single chain antibodies are formed by linking the Fv regions from a heavy and a light chain via an amino acid bridge, resulting in a single chain polypeptide that folds into an antigen-binding site.

Antigen binding fragments of antibodies that specifically recognize peptides according to the present invention (e.g., SEQ. ID Nos:1 through 27) may also be generated using methods that are well known in the art and are also useful for therapeutic and diagnostic applications as described herein. For example, such fragments include but are not limited to: the F(ab')2 fragments which can be produced by art-known methods such as pepsin digestion of the antibody molecule and the Fab fragments which can be generated by art-known methods such as reducing the disulfide bridges of the F(ab')2 fragments. Fab expression libraries may be constructed to allow rapid and easy identification of monoclonal Fab fragments having specificity for peptides according to the present invention. (Huse et al., Science 246(4935):1275-81 (1989)). The ordinary skilled practitioner will appreciate that there are many useful variations of the above-described methods.

The polypeptides and antibodies of the invention can be used in methods of diagnosing SARS. This may be done utilizing such procedures as enzyme linked immunosorbant assay (ELISA), Western Blots, immunoprecipitation and immunofluorescence. A test sample from an individual may be assessed for the presence of a SARS-CoV polypeptide. In one embodiment, an antibody capable of binding to the polypeptide (as described above) can be used. Preferably, the antibody contains a detectable label. An intact antibody or antigen-binding fragment thereof may be used. Detection assays may utilize direct and/or indirect labels. For example, but not by way of limitation, the presence or absence of the polypeptide of interest (e.g., a polypeptide of the invention or a variant or fragment thereof) in a sample can be assessed by contacting the sample with an antibody that specifically binds the polypeptide of interest. The antibody is preferably labeled using any suitable detectable label, of which many are known in the art. The sample is then assessed for the presence or absence of binding of the antibody. The presence of the polypeptide of the invention is diagnostic for SARS-CoV infection. In an additional embodiment, the peptides of the invention can be immobilized on latex particles for purposes of producing a serological passive agglutination test. This test has clinical significance for identifying recent infection (IgM), previous exposure and infection (IgG), state of current infection (prognosis) and state of patient immunity (various immunoglobulins). In these tests, polypeptides of the present invention are used to bind to, and thereby detect immunoglobulins in a subject's body fluids or tissues. In such tests, the polypeptides of the present invention are preferably labeled with any suitable detectable label.

The methods of the present invention may be employed, for example, but not by way of limitation, to test clinical specimens obtained from suspected SARS patients. The specimens, or test samples, may be collected from any source suspected of containing SARS viral proteins. For animals, preferably, mammals, and more preferably, humans, the source of the test samples may include blood, bone marrow, lymph, hard tissues (e.g., liver, spleen, kidney, lung, ovary, etc.), sputum, feces, urine, upper and lower respiratory specimens and other clinical samples. Other sources may include veterinary and environmental samples, as well as *in vitro* cultures. Those skilled in the art are capable of determining appropriate clinical sources for use in diagnosis of SARS-CoV infection.

The compositions of the present invention are also useful for therapeutic purposes. The present invention also provides pharmaceutical compositions comprising the polypeptides described (e.g., SEQ ID NOs:1 through 27) and methods of treatment of SARS infection using a therapeutic agent. The polypeptides, fragments or variants of the invention may be prepared with a carrier (i.e., water, salt solution, saline, alcohols, glycerol and other compositions known to one with skill in the art), producing a therapeutic agent. A SARS-CoV therapeutic agent is an agent that alters SARS-CoV polypeptide activity or expression by, for example, but not by way of limitation, upregulating or downregulating gene transcription or translation or by interfering with SARS-CoV polypeptide activity. SARS-CoV therapeutic agents can include the polypeptides of the invention (e.g., SEQ ID NOs:1 through 27). The carrier may be in the form of a liquid, suspension, pill, tablet, powder and other form known to one with skill in the art.

The preparations of the present invention are administered in effective amounts. An effective amount is that amount of a pharmaceutical preparation that alone, or together with further doses, produces the desired response. The desired response may be, for example, prevention of infection, reduction of viral load, improvement in immune response, or decrease in morbidity and/or mortality. The effective amount will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. It is preferred generally that a maximum dose be used, that is, the highest safe dose according to sound medical judgment. A skilled practitioner can determine the amount that will be therapeutically effective by standard clinical techniques.

When administered, the pharmaceutical preparations of the invention are applied in pharmaceutically-acceptable amounts and in pharmaceutically-acceptably compositions. Such preparations may routinely contain salts, buffering agents, preservatives, compatible carriers, and optionally, other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, phannaceutically-acceptable salts can be prepared as alkaline metal or alkaline earth salts, such as sodium, potassium or calcium salts.

The SARS-CoV polypeptides useful according to the present invention may be combined, optionally, with a pharmaceutically acceptable carrier. The term "pharmaceutically-acceptable carrier," as used herein, means one or more compatible solid or liquid fillers, diluents or encapsulating substances that are suitable for administration into a human. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction that would substantially impair the desired pharmaceutical efficacy. The pharmaceutical compositions may contain suitable buffering agents, including acetic acid in a salt; citric acid in a salt; and phosphoric acid in a salt. The pharmaceutical compositions also may contain, optionally, suitable preservatives, such as benzalkonium chloride; chlorobutanol; parabens and thimerosal.

The pharmaceutical compositions may conveniently be presented in unit dosage form and may be prepared by any of the methods well known in the art of pharmacy. Most methods include the step of bringing the active agent into association with a carrier that constitutes one or more accessory ingredients. In general, the compositions are prepared by uniformly and intimately bringing the active compound into association with a liquid carrier, a finely divided solid carrier, or both, and then, if necessary, shaping the product.

The polypeptides and antibodies of the invention may also be combined with other reagents and articles to form kits. Diagnostic kits often contain the polypeptide and/or antibody and other reagents to perform hybridization reactions (such as medias, salts, buffers, etc.). The kit may also contain reagents for sample processing, immobilization materials, labeling materials and positive/negative controls or reference samples, and the like. The invention also provides a pack or kit comprising one or more containers filled with one or more of the ingredients used in the present invention. The kit can comprise a single unit use of the compositions, or it can comprise enough of the compositions that the reactions or assays may be performed a plurality of times. The agents in the kit can be separated, mixed together in any combination, or present in a single vial.

The following experimental examples are provided to illustrate certain embodiments of the invention, but are not intended to limit the invention.

### EXAMPLE 1

The N proteins of human coronaviruses are purported to be relatively conserved, type-specific, and abundant. The SARS-CoV N protein was, therefore, targeted as a starting point for identifying conserved, specific, and antigenic peptides suitable for use as immunogens or ligands for traditional or non-traditional antibody development.

Published protein sequences of human coronavirus were compared using commercial software in hopes of identifying proteins or peptides that could potentially serve directly or indirectly as important biologicals in the diagnosis or treatment of SARS-CoV related illness. Nucleocapsid protein (N) sequences 229E, OC43 and SARS-CoV were compared, and the data suggested that there was enough intra-virus N sequence homology and inter-virus N sequence divergency to afford the opportunity to identify conserved and specific SARS-CoV polypeptides. The SARS-CoV N protein sequence was analyzed using antigenicity algorithms, and potentially-antigenic peptides were identified. Data from a Basic Local Alignment Search Tool (BLAST) query performed on five selected SARS-CoV N antigenic peptides suggested there is insignificant homology with non-SARS-CoV N peptides in the database. Therefore, these five conserved SARS-CoV N specific peptides, because of their inherent specificity and antigenicity, may, therefore, serve as the primary component in a prepared immunogen intended to induce an immunogenic response in animals for purposes of producing SARS-CoV N reactive monoclonal or polyclonal antibodies. Alternatively, these peptides could serve as the primary component in an immobilized ligand intended to pan either an antibody or antibody component phage display library with the intention of selecting phage clones that could be manipulated to produce a SARS-CoV N reactive antibody producing prokaryotic or eukaryotic cell.

Using Omiga, or DS Gene software, N protein sequences of SARS-CoV were analyzed using (1) Hopp/Woods, (2) Parker, (3) protrusion index, and (4) Welling antigenicity algorithms.

The Hopp and Woods method predicts the location of antigenic determinants by finding the area of greatest local hydrophobicity.

The Parker method is based on the Hopp and Woods method. It predicts the location of antigenic determinants by finding the area of greatest local hydrophobicity. This method differs from the Hopp and Woods method in that this method uses a modified hydrophobicity scale, based on the HPLC retention times of model peptides.

The Protrusion method uses the Protrusion Index, which is an antigenic scale based on a study of proteins with known 3D structure. The tendency of each residue to be located in a protruding region of the protein can be calculated using this method.

The Welling method calculates a statistical score, where the antigenicity value for each residue is calculated as the log of the quotient between its percentage in a sample of known antigenic regions and its percentage in average proteins.

Consequently, antigenicity plots were generated, and the five common antigenic domains or peptides of the N protein listed in Table 1 were identified.

**Table 1 Potentially Antigenic SARS-CoV Nucleocapsid Peptides and Protein Domains**

| **REGION** | **SEQ. #** | **SEQUENCE** | **SEQ ID NO.** |
|---|---|---|---|
| **SARS-CoV Nucleocapsid (N) Protein** | | | |
| | 1 | Gly-Pro-Thr-Asp-Ser-Thr- Asp-Asn-Asn-Gln-Asn-Gly-Gly- Arg-Asn-Gly-Ala-Arg-Pro-Lys-Gln- Arg-Arg-Pro-Gln | SEQ ID NO: 1 |
| | 2 | Pro-Ile-Asn-Thr-Asn-Ser- Gly-Pro-Asp-Asp-Gln-Ile-Gly-Tyr- Tyr-Arg-Arg-Ala-Thr-Arg-Arg-Val- Arg-Gly-Gly-Asp-Gly | SEQ ID NO:2 |
| | 3 | Gly-Ser-Arg-Gly-Gly-Ser- Gln-Ala-Ser-Ser-Arg-Ser-Ser-Ser- Arg-Ser-Arg-Gly-Asn-Ser- Arg-Asn- Ser-Thr-Pro-Gly-Ser-Ser-Arg-Gly- Asn-Ser-Pro-Ala-Arg-Met-Ala | SEQ ID NO:3 |
| | 4 | Lys-Val-Ser-Gly-Lys-Gly- Gln-Gln-Gln-Gln-Gly-Gln-Thr-Val- Thr-Lys-Lys-Ser-Ala-Ala-Glu-Ala- Ser-Lys-Lys-Pro-Arg-Gln-Lys-Arg- Thr-Ala-Thr-Lys-Gln-Tyr-Asn | SEQ ID NO:4 |
| | 5 | Tyr-Lys-Thr-Phe-Pro-Pro- Thr-Glu-Pro-Lys-Lys-Asp-Lys-Lys- Lys-Lys-Thr-Asp-Glu-Ala-Gln-Pro- Leu-Pro-Gln-Arg-Gln | SEQ ID NO:5 |

A small peptide BLAST query on each individual peptide was performed to identify potential homologs in the National Center for Biotechnology Information (NCBI) database. It is inferred from the data that identified homologs are divergent enough to pose a low risk for potentially causing specificity or false positivity issues with antibodies generated from these peptides.

### EXAMPLE 2

Using the same strategy, additional SARS-CoV proteins were analyzed, and potentially antigenic peptides were identified.

In particular, the thirteen Spike (S), two Envelope (E) and seven Matrix (M) peptides listed in Table 2 were selected.

**Table 2 Potentially Antigenic SARS-CoV Spike, Envelope and Matrix Peptides and Protein Domains**

| **REGION** | **SEQ. #** | **SEQUENCE** | **SEQ ID NO.** |
|---|---|---|---|
| **SARS-CoV Spike (S) Protein** | | | |
| | 1 | Ser-Gly-Ser-Asp-Leu-Asp- Arg-Cys-Thr-Thr-Phe-Asp-Asp-Val- Gln-Ala-Pro-Asn-Tyr-Thr-Gly-His- Thr-Ser-Ser-Met-Arg-Gly-Val-Tyr- Tyr-Pro-Asp-Glu-Ile-Phe-Arg-Ser- Asp-Thr-Leu-Tyr | SEQ ID NO:6 |
| | 2 | Ser-Asp-Ala-Phe-Ser-Leu- Asp-Val-Ser-Glu-Lys-Ser-Gly-Asn- Phe-Lys-His-Leu-Arg-Glu-Phe-Val- Phe-Lys-Asn | SEQ ID NO:7 |
| | 3 | Glu-Asn-Gly-Thr-Ile-Thr- Asp-Ala-Val-Asp-Cys-Ser-Gln-Asn- Pro-Leu-Ala-Glu-Leu-Lys-Cys-Ser- Val-Lys-Ser-Phe-Glu-Ile-Asp-Lys | SEQ ID NO:8 |
| | 4 | Thr-Ala-Trp-Glu-Arg-Lys- Lys-Ile-Ser-Asn-Cys-Val | SEQ ID NO:9 |
| | 5 | Ser-Phe-Val-Val-Lys-Gly- Asp-Asp-Val-Arg-Gln-Ile-Ala | SEQ ID NO:10 |
| | 6 | Tyr-Arg-Tyr-Leu-Arg-His- Gly-Lys-Leu-Arg-Pro-Phe-Glu-Arg- Asp-Ile-Ser-Asn-Val-Pro-Phe-Ser- Pro-Asp-Gly-Lys-Pro-Cys-Thr | SEQ ID NO:11 |
| | 7 | Val-Leu-Thr-Pro-Ser-Ser- Lys-Arg-Phe-Gln-Pro | SEQ ID NO:12 |
| | 8 | Gln-Phe-Gly-Arg-Asp-Val- Ser-Asp-Phe-Thr-Asp-Ser-Val-Arg- Asp-Pro-Lys-Thr-Ser-Glu-Ile-Leu- Asp-Ile | SEQIDNO:13 |
| | 9 | Leu-Ser-Gly-Ile-Ala-Ala- Glu-Gln-Asp-Arg-Asn-Thr-Arg- Glu-Val-Phe-Ala | SEQIDNO:14 |
| | 10 | Leu-Pro-Asp-Pro-Leu-Lys- Pro-Thr-Lys-Arg-Ser-Phe-Ile-Glu | SEQ ID NO:15 |
| | 11 | Leu-Asn-Asp-Ile-Leu-Ser- Arg-Leu-Asp-Lys-Val-Glu-Ala-Glu- Val-Gln | SEQ ID NO:16 |
| | 12 | Pro-Leu-Gln-Pro-Glu-Leu- Asp-Ser-Phe-Lys-Glu-Glu-Leu-Asp- Lys-Thr-Phe-Lys-Asn-His-Thr-Ser- Pro-Asp-Val-Asp-Ile-Gly-Asp-Ile- Ser-Gly-Ile | SEQ ID NO:17 |
| | 13 | Cys-Cys-Ser-Cys-Leu-Lys- Gly-Ala-Cys-Ser-Cys-Gly-Ser-Cys- Cys-Lys-Phe-Asp-Glu-Asp-Asp-Ser- Glu-Pro-Val-Leu-Lys-Gly-Val | SEQIDNO:18 |
| **SARS-CoV Envelope (E) Protein** | | | |
| | 1 | Val-Ser-Glu-Glu-Thr-Gly- Thr-Leu-Ile-Val-Asn-Ser | SEQ ID NO:19 |
| | 2 | Ser-Arg-Val-Lys-Asn-Leu- Asn-Ser-Ser-Glu-Gly-Val | SEQ ID NO:20 |
| **SARS-CoV Matrix (M) Protein** | | | |
| | 1 | Gly-Thr-Ile-Thr-Val-Glu- Glu-Leu-Lys-Gln-Leu-Leu-Glu-Gln- Trp-Asn | SEQ ID NO:21 |
| | 2 | Leu-Leu-Gln-Phe-Ala-Tyr- Ser-Asn-Arg-Asn-Arg-Phe-Leu-Tyr- Ile-Ile | SEQ ID NO:22 |
| | 3 | Tyr-Phe-Val-Ala-Ser-Phe- Arg-Leu-Phe-Ala-Arg-Thr-Arg-Ser- Met-Trp-Ser-Phe-Asn | SEQ ID NO:23 |
| | 4 | Arg-Gly-Thr-Ile-Val-Thr- Arg-Pro-Leu-Met-Glu-Ser-Glu-Leu- Val-Ile | SEQ ID NO:24 |
| | 5 | Leu-Gly-Arg-Cys-Asp-Ile- Lys-Asp-Leu-Pro-Lys-Glu-Ile-Thr- Val-Ala | SEQ ID NO:25 |
| | 6 | Gly-Ala-Ser-Gln-Arg-Val- Gly-Thr-Asp-Ser | SEQ ID NO:26 |
| | 7 | Asn-Tyr-Lys-Leu-Asn-Thr- Asp-His-Ala-Gly-Ser-Asn | SEQ ID NO:27 |

The foregoing presentation of the described embodiments is provided to enable any person skilled in the art to make or use the present invention. Various modifications to these embodiments are possible, and the generic principles presented herein may be applied to other embodiments as well.

The abstract is not to be construed as limiting the scope of the present invention, as its purpose is to enable the appropriate authorities, as well as the general public, to quickly determine the general nature of the invention.

An isolated polypeptide encoded by a SARS-CoV gene, or a fragment or variant of said polypeptide, wherein said polypeptide comprises an amino acid sequence selected from the group consisting of SEQ ID NOs: 1 through 27.

The isolated polypeptide as defined above, wherein the polypeptide is produced by recombinant means.

An isolated polypeptide comprising an amino acid sequence which is greater than about 90% identical to an amino acid sequence selected from SEQ ID NOs:1 through 27.

A fusion protein comprising the isolated polypeptide as defined above.

An antibody, or an antigen-binding fragment thereof, that selectively binds to an amino acid sequence selected from the group consisting of SEQ ID NOs:1 through 27, or to a fragment or variant of the amino acid sequence.

The antibody as defined above, wherein the antibody is a monoclonal antibody.

A method of diagnosing SARS comprising detecting a polypeptide as defined above in a test sample, wherein the presence of the polypeptide is indicative of the presence of SARS in the sample.

The method as defined above, wherein the method comprises contacting the test sample with an antibody specific for the polypeptide.

A method for assaying for the presence of the polypeptide as defined above in a sample, comprising contacting the sample with an antibody which specifically binds to the polypeptide and detecting the presence of the bound antibody.

A pharmaceutical composition, comprising the polypeptide as defined above, and a pharmaceutically acceptable carrier.

A method for the treatment of SARS, wherein the method comprising administering an effective amount of the pharmaceutical composition as defined above to a patient in need thereof.

A kit, comprising: a) at least one antibody which selectively binds to an amino acid sequence selected from the group consisting of SEQ ID NOs: 1 through 27; and b) a reference protein sample.

A kit, comprising a) at least one isolated polypeptide selected from the group consisting of SEQ ID NOs:1 through 27; and b) a reference protein sample.

A method of identifying an agent which alters activity of the polypeptide as defined above, comprising:
a) contacting the polypeptide or fragment thereof with an agent to be tested;
b) assessing the level of activity of the polypeptide or fragment thereof; and
c) comparing the level of activity with the level of activity of the polypeptide or fragment thereof in the absence of the agent;
wherein if the level of activity of the polypeptide or fragment thereof in the presence of the agent differs from the level in the absence of the agent, then the agent is an agent that alters activity of the peptide.

An agent which alters the activity of the polypeptide as defined above, identifiable according to the method as defined above.

## Claims

1. An isolated polypeptide encoded by a SARS-CoV gene, or a fragment or variant of said polypeptide, wherein said polypeptide comprises an amino acid sequence selected from the group consisting of SEQ ID NOs:5, 2, 3, 4 and 6 through 27.

2. The isolated polypeptide of claim 1, wherein the polypeptide is produced by recombinant means.

3. An isolated polypeptide comprising an amino acid sequence which is greater than about 90% identical to an amino acid sequence selected from SEQ ID NOs:5, 2, 3, 4 and 6 through 27.

4. A fusion protein comprising the isolated polypeptide of claim 1.

5. An antibody, or an antigen-binding fragment thereof, that selectively binds to an amino acid sequence selected from the group consisting of SEQ ID NOs:5, 2, 3, 4 and 6 through 27, or to a fragment or variant of the amino acid sequence.

6. The antibody of claim 5, wherein the antibody is a monoclonal antibody.

7. A method of diagnosing SARS comprising detecting a polypeptide of claim 1 in a test sample, wherein the presence of the polypeptide is indicative of the presence of SARS in the sample.

8. The method of claim 7, wherein the method comprises contacting the test sample with an antibody specific for the polypeptide.

9. A method for assaying for the presence of the polypeptide of claim 1 in a sample, comprising contacting the sample with an antibody which specifically binds to the polypeptide and detecting the presence of the bound antibody.

10. A pharmaceutical composition, comprising the polypeptide of claim 1, and a pharmaceutically acceptable carrier.

11. A method for the treatment of SARS, wherein the method comprising administering an effective amount of the pharmaceutical composition of claim 10 to a patient in need thereof.

12. A kit, comprising: a) at least one antibody which selectively binds to an amino acid sequence selected from the group consisting of SEQ ID NOs:5, 2, 3, 4 and 6 through 27; and b) a reference protein sample.

13. A kit, comprising a) at least one isolated polypeptide selected from the group consisting of SEQ ID NOs:5, 2, 3, 4 and 6 through 27; and b) a reference protein sample.

14. A method of identifying an agent which alters activity of the polypeptide of claim 1, comprising:
a) contacting the polypeptide or fragment thereof with an agent to be tested;
b) assessing the level of activity of the polypeptide or fragment thereof; and
c) comparing the level of activity with the level of activity of the polypeptide or fragment thereof in the absence of the agent;
wherein if the level of activity of the polypeptide or fragment thereof in the presence of the agent differs from the level in the absence of the agent, then the agent is an agent that alters activity of the peptide.

15. An agent which alters the activity of the polypeptide of claim 1, identifiable according to the method of claim 14.


SEQUENCE LISTING

<110> BECTON, DICKINSON AND COMPANY

<120> IMMUNOGENIC SARS DOMAIN

<130> P-6196.70

<140>
<141>

<150> 60/580,098
<151> 2004-06-17

<160> 27

<170> PatentIn Ver. 3.3

<210> 1
<211> 25
<212> PRT
<213> SARS coronavirus

<400> 1
Gly Pro Thr Asp Ser Thr Asp Asn Asn Gln Asn Gly Gly Arg Asn Gly
  1               5                  10                  15

Ala Arg Pro Lys Gln Arg Arg Pro Gln
             20                  25

<210> 2
<211> 27
<212> PRT
<213> SARS coronavirus

<400> 2
Pro Ile Asn Thr Asn Ser Gly Pro Asp Asp Gln Ile Gly Tyr Tyr Arg
  1               5                  10                  15

Arg Ala Thr Arg Arg Val Arg Gly Gly Asp Gly
             20                  25

<210> 3
<211> 37
<212> PRT
<213> SARS coronavirus

<400> 3
Gly Ser Arg Gly Gly Ser Gln Ala Ser Ser Arg Ser Ser Ser Arg Ser
  1               5                  10                  15

Arg Gly Asn Ser Arg Asn Ser Thr Pro Gly Ser Ser Arg Gly Asn Ser
             20                  25                  30

Pro Ala Arg Met Ala
         35

<210> 4
<211> 37

<212> PRT
<213> SARS coronavirus

<400> 4
Lys Val Ser Gly Lys Gly Gln Gln Gln Gln Gly Gln Thr Val Thr Lys
1               5                   10                  15

Lys Ser Ala Ala Glu Ala Ser Lys Lys Pro Arg Gln Lys Arg Thr Ala
            20                  25                  30

Thr Lys Gln Tyr Asn
        35


<210> 5
<211> 27
<212> PRT
<213> SARS coronavirus

<400> 5
Tyr Lys Thr Phe Pro Pro Thr Glu Pro Lys Lys Asp Lys Lys Lys Lys
1               5                   10                  15

Thr Asp Glu Ala Gln Pro Leu Pro Gln Arg Gln
            20                  25


<210> 6
<211> 42
<212> PRT
<213> SARS coronavirus

<400> 6
Ser Gly Ser Asp Leu Asp Arg Cys Thr Thr Phe Asp Asp Val Gln Ala
1               5                   10                  15

Pro Asn Tyr Thr Gly His Thr Ser Ser Met Arg Gly Val Tyr Tyr Pro
            20                  25                  30

Asp Glu Ile Phe Arg Ser Asp Thr Leu Tyr
        35                  40


<210> 7
<211> 25
<212> PRT
<213> SARS coronavirus

<400> 7
Ser Asp Ala Phe Ser Leu Asp Val Ser Glu Lys Ser Gly Asn Phe Lys
1               5                   10                  15

His Leu Arg Glu Phe Val Phe Lys Asn
            20                  25


<210> 8
<211> 30
<212> PRT
<213> SARS coronavirus

<400> 8
Glu Asn Gly Thr Ile Thr Asp Ala Val Asp Cys Ser Gln Asn Pro Leu

1               5                   10                  15

Ala Glu Leu Lys Cys Ser Val Lys Ser Phe Glu Ile Asp Lys
            20                  25                  30


<210> 9
<211> 12
<212> PRT
<213> SARS coronavirus

<400> 9
Thr Ala Trp Glu Arg Lys Lys Ile Ser Asn Cys Val
1               5                   10


<210> 10
<211> 13
<212> PRT
<213> SARS coronavirus

<400> 10
Ser Phe Val Val Lys Gly Asp Asp Val Arg Gln Ile Ala
1               5                   10


<210> 11
<211> 29
<212> PRT
<213> SARS coronavirus

<400> 11
Tyr Arg Tyr Leu Arg His Gly Lys Leu Arg Pro Phe Glu Arg Asp Ile
1               5                   10                  15

Ser Asn Val Pro Phe Ser Pro Asp Gly Lys Pro Cys Thr
            20                  25


<210> 12
<211> 11
<212> PRT
<213> SARS coronavirus

<400> 12
Val Leu Thr Pro Ser Ser Lys Arg Phe Gln Pro
1               5                   10


<210> 13
<211> 24
<212> PRT
<213> SARS coronavirus

<400> 13
Gln Phe Gly Arg Asp Val Ser Asp Phe Thr Asp Ser Val Arg Asp Pro
1               5                   10                  15

Lys Thr Ser Glu Ile Leu Asp Ile
            20


<210> 14

<211> 17
<212> PRT
<213> SARS coronavirus

<400> 14
Leu Ser Gly Ile Ala Ala Glu Gln Asp Arg Asn Thr Arg Glu Val Phe
1               5                   10                  15

Ala

<210> 15
<211> 14
<212> PRT
<213> SARS coronavirus

<400> 15
Leu Pro Asp Pro Leu Lys Pro Thr Lys Arg Ser Phe Ile Glu
1               5                   10

<210> 16
<211> 16
<212> PRT
<213> SARS coronavirus

<400> 16
Leu Asn Asp Ile Leu Ser Arg Leu Asp Lys Val Glu Ala Glu Val Gln
1               5                   10                  15

<210> 17
<211> 33
<212> PRT
<213> SARS coronavirus

<400> 17
Pro Leu Gln Pro Glu Leu Asp Ser Phe Lys Glu Glu Leu Asp Lys Thr
1               5                   10                  15

Phe Lys Asn His Thr Ser Pro Asp Val Asp Ile Gly Asp Ile Ser Gly
            20                  25                  30

Ile

<210> 18
<211> 29
<212> PRT
<213> SARS coronavirus

<400> 18
Cys Cys Ser Cys Leu Lys Gly Ala Cys Ser Cys Gly Ser Cys Cys Lys
1               5                   10                  15

Phe Asp Glu Asp Asp Ser Glu Pro Val Leu Lys Gly Val
            20                  25

<210> 19
<211> 12

<212> PRT
<213> SARS coronavirus

<400> 19
Val Ser Glu Glu Thr Gly Thr Leu Ile Val Asn Ser
1               5                   10


<210> 20
<211> 12
<212> PRT
<213> SARS coronavirus

<400> 20
Ser Arg Val Lys Asn Leu Asn Ser Ser Glu Gly Val
1               5                   10


<210> 21
<211> 16
<212> PRT
<213> SARS coronavirus

<400> 21
Gly Thr Ile Thr Val Glu Glu Leu Lys Gln Leu Leu Glu Gln Trp Asn
1               5                   10                  15


<210> 22
<211> 16
<212> PRT
<213> SARS coronavirus

<400> 22
Leu Leu Gln Phe Ala Tyr Ser Asn Arg Asn Arg Phe Leu Tyr Ile Ile
1               5                   10                  15


<210> 23
<211> 19
<212> PRT
<213> SARS coronavirus

<400> 23
Tyr Phe Val Ala Ser Phe Arg Leu Phe Ala Arg Thr Arg Ser Met Trp
1               5                   10                  15

Ser Phe Asn


<210> 24
<211> 16
<212> PRT
<213> SARS coronavirus

<400> 24
Arg Gly Thr Ile Val Thr Arg Pro Leu Met Glu Ser Glu Leu Val Ile
1               5                   10                  15


<210> 25
<211> 16

<212> PRT
<213> SARS coronavirus

<400> 25
Leu Gly Arg Cys Asp Ile Lys Asp Leu Pro Lys Glu Ile Thr Val Ala
1               5                   10                  15


<210> 26
<211> 10
<212> PRT
<213> SARS coronavirus

<400> 26
Gly Ala Ser Gln Arg Val Gly Thr Asp Ser
1               5                   10


<210> 27
<211> 12
<212> PRT
<213> SARS coronavirus

<400> 27
Asn Tyr Lys Leu Asn Thr Asp His Ala Gly Ser Asn
1               5                   10